# FASCICULE DE BREVET EUROPEEN

(11) **EP 3 472 094 B1**
(45) Date de publication et mention de la délivrance du brevet: **22.03.2023**
(21) Numéro de dépôt: 17742804.2
(22) Date de dépôt: 20.06.2017
(51) Int. Cl.: B67D 7/34, G01N 33/28

(54) **BARRIÈRE TECHNIQUE ET PROCÉDÉ DE SÉCURISATION DE DÉPOTAGES**
TECHNISCHE BARRIERE UND VERFAHREN ZUR GEWÄHRLEISTUNG DER SICHERHEIT VON ENTLADUNGSOPERATIONEN
ENGINEERED BARRIER AND METHOD OF ENSURING THE SECURITY OF DISCHARGING OPERATIONS

(30) Priorité: 20.06.2016 FR 1655708
(43) Date de publication de la demande: 24.04.2019
(73) Titulaire: Veolia Environnement, 75008 Paris (FR)
(72) Inventeur: BENANOU, David, 78500 Sartrouville (FR)
(74) Mandataire: Cabinet Beau de Loménie
(86) Numéro de dépôt international: PCT/FR2017/051615
(87) Numéro de publication internationale: WO 2017/220915

(56) Documents cités:
- EP-A1- 2 474 500
- WO-A1-98/20342
- JP-A- S59 102 629
- US-A- 5 209 275
- US-A- 5 722 469
- US-A1- 2014 129 038

## Description

### DOMAINE DE L'INVENTION

Le présent exposé concerne une barrière technique pour la sécurisation de dépotages ainsi qu'un procédé de sécurisation de dépotages permettant de vérifier la nature de la matière à dépoter afin de prévenir un éventuel accident de dépotage.

Une telle barrière ou procédé peut être utilisé notamment sur des sites industriels pour réduire le risque d'erreur de dépotage et donc le risque de formation d'un mélange incompatible de deux matières chimiques pouvant entraîner une explosion ou un dégagement de gaz toxiques.

### ETAT DE LA TECHNIQUE ANTERIEURE

La sécurisation des dépotages est une problématique majeure dans la gestion des produits chimiques dangereux. En effet, certaines erreurs de dépotage peuvent conduire à dépoter un produit non attendu dans une cuve de stockage comprenant un produit différent, potentiellement incompatible : dans un tel cas, le mélange de ces produits incompatibles peut entraîner des réactions chimiques non souhaitées potentiellement dangereuses, entraînant par exemple un échauffement excessif, avec des dégagements de gaz toxiques exposant le personnel d'exploitation du site et les populations environnantes sur plusieurs kilomètres autour du site.

Le cas échéant, le produit initialement présent dans une cuve de stockage sera à minima dilué, dégradé ou contaminé. De telles erreurs de dépotage entraînent donc des conséquences sanitaires, financières et/ou environnementales importantes, potentiellement catastrophiques, qu'il convient donc de maîtriser.

A cette fin, de nombreuses mesures de sécurisation existent mais la plupart restent des mesures organisationnelles. Par exemple, on peut citer le contrôle des documents de livraison de la matière à dépoter et de la signalétique des cuves du site de livraison ou encore le respect d'un protocole strict de livraison ; les livraisons peuvent également être organisées de manière à ne pas dépoter de matières incompatibles le même jour. Toutefois, ces mesures mettent en oeuvre un facteur humain, source d'erreur, et les mesures mises en place sur le site de livraison sont impuissantes face à des erreurs survenues sur le site de chargement conduisant à la présence d'une mauvaise matière dans le camion de livraison.

D'autres mesures peuvent être des mesures techniques et mettent en oeuvre des capteurs de pression, de température ou de gaz toxiques tels que le Cl2 ou le SO2 pour détecter les conséquences d'un mélange incompatible et déclencher dans un tel cas une alarme ainsi que l'interruption automatique du dépotage. Toutefois, de telles mesures détectent un gaz toxique lorsqu'il a déjà commencé à se former. Ainsi, le mélange incompatible dans la cuve de stockage réceptrice a déjà eu lieu. En outre, dans le cas d'une réaction chimique à cinétique rapide, la détection peut ne pas survenir suffisamment tôt pour empêcher le dégagement de gaz toxique redouté.

Parmi les systèmes de sécurisation connus, on connaît également celui du document WO 98/20342.

Il existe donc un réel besoin pour une barrière technique pour la sécurisation de dépotages ainsi qu'un procédé de sécurisation de dépotages qui soient dépourvus, au moins en partie, des inconvénients inhérents aux mesures connues précitées.

### PRESENTATION DE L'INVENTION

Le présent exposé concerne une barrière technique pour la sécurisation de dépotages, comprenant un élément de barrage configuré pour bloquer ou laisser passer une matière à dépoter en fonction d'une commande qui lui est adressée, un élément de mesure configuré pour mesurer au moins un paramètre de la matière à dépoter, un module de validation configuré pour comparer au moins un paramètre mesuré par ledit élément de mesure avec un paramètre théorique préenregistré pour la matière attendue pour le dépotage, et déterminer, sur la base de cette comparaison, si la matière à dépoter correspond à la matière attendue pour le dépotage, et un module de commande configuré pour commander l'élément de barrage en fonction des résultats de cette détermination.

Ainsi, grâce à une telle barrière technique, il est possible de s'assurer que la matière à dépoter est bien la matière attendue pour le dépotage de telle sorte que le dépotage ne risque pas d'entraîner un mélange non souhaité et éventuellement incompatible provoquant un dégagement de gaz toxique ou la contamination d'une cuve de stockage avec un produit non souhaité.

On renforce ainsi la sécurité du site et des populations aux alentours en réduisant, voire en supprimant complètement le risque d'explosion ou de dégagement de gaz toxiques notamment.

En outre, une telle barrière technique peut être totalement automatisée de telle sorte qu'elle n'est pas sensible à d'éventuelles erreurs humaines : elle empêchera ainsi le dépotage même si un technicien se trompe de cuve ou ne remarque pas une incohérence dans les documents de livraison.

De plus, elle contrôle la nature même de la matière à dépoter et pourra donc empêcher le dépotage même si les documents de livraison sont erronés ou qu'une matière différente a été chargée par erreur dans le camion de livraison.

En outre, ceci permet d'exercer un contrôle en amont, avant le début du dépotage, de telle sorte qu'aucun mélange incompatible ne peut se produire, même en très petite quantité. Ainsi, elle permet de signaler une éventuelle erreur mais également de prévenir les dommages qui seraient causés par une erreur de dépotage.

Dans certains modes de réalisation, l'élément de mesure est un analyseur en ligne configuré pour mesurer au moins un paramètre de la matière à dépoter sans prélèvement de matière. Grâce à un tel analyseur en ligne, il est possible d'effectuer la mesure in situ et en temps réel sans devoir prélever de la matière, éventuellement dangereuse, et l'analyser dans un laboratoire séparé, retardant les opérations de dépotage. De plus, un tel analyseur en ligne permet de renouveler le cas échéant la mesure au cours du dépotage.

Dans certains modes de réalisation, l'analyseur en ligne est configuré pour mesurer au moins un paramètre de la matière à dépoter sans contact avec ladite matière à dépoter. La matière à dépoter pouvant être dangereuse, corrosive par exemple, on évite de cette manière que la matière à dépoter ne puisse endommager l'analyseur, ce qui prolonge sa durée de vie. On évite également la dérive de l'analyseur permettant ainsi une diminution du cout de maintenance de l'analyseur.

Dans certains modes de réalisation, l'élément de mesure est un spectromètre, de préférence de type Raman. La mesure d'un spectre, qu'il soit Raman, infra-rouge, RMN ou d'une autre nature, permet d'obtenir une caractérisation précise de la matière analysée, chaque substance chimique ayant un spectre qui lui est propre. Ceci renforce la fiabilité de la barrière technique. La spectroscopie Raman est préférée, notamment par rapport à la spectroscopie infra-rouge, car elle permet d'identifier facilement une substance chimique même lorsque celle-ci est diluée, dans de l'eau notamment.

Toutefois, l'élément de mesure peut naturellement être d'un autre type et mesurer un ou plusieurs autres paramètres de la matière à dépoter, notamment son pH ou sa densité.

Dans certains modes de réalisation, la barrière technique comprend un regard de coulée configuré pour recevoir la matière à dépoter en amont de l'élément de barrage, le regard de coulée possédant au moins une fenêtre transparente au niveau de laquelle, de préférence contre laquelle, est prévu l'élément de mesure. Un tel regard de coulée permet ainsi d'observer la matière à dépoter juste en amont de l'élément de barrage : il est ainsi possible de mettre en place l'élément de mesure au niveau du regard de coulée pour avoir facilement accès à la matière à analyser. Un tel regard de coulée est ainsi particulièrement utile lorsque l'élément de mesure est un spectromètre.

Dans certains modes de réalisation, le regard de coulée comprend un conduit en matériau transparent, de préférence en verre.

Dans certains modes de réalisation, la barrière technique comprend une vanne de vidange prévue en aval du regard de coulée et en amont de l'élément de barrage. Ceci permet de réduire le risque qu'un matière issue d'un dépotage antérieur ne reste dans le regard de coulée et ne perturbe la mesure de l'élément de mesure.

Dans certains modes de réalisation, la barrière technique comprend une vanne d'isolement prévue entre le regard de coulée et l'élément de barrage.

Dans certains modes de réalisation, la barrière technique comprend un module de vérification de vidange configuré pour vérifier que la vidange du regard de coulée est effective et pour interdire une opération de dépotage ultérieure si cette vérification échoue. Ceci permet de s'assurer que le regard de coulée est bien vide avant d'entamer une nouvelle opération de dépotage et donc de s'assurer que la mesure effectuée par l'élément de mesure concerne bien la matière à dépoter de l'opération de dépotage en cours.

Dans certains modes de réalisation, la barrière technique comprend une enceinte d'isolation thermique englobant au moins ledit élément de mesure afin de l'isoler du milieu environnant. Une telle enceinte d'isolation thermique permet de protéger du froid l'élément de mesure afin d'assurer un bon fonctionnement et donc une bonne fiabilité de ce dernier.

Dans certains modes de réalisation, l'enceinte d'isolation thermique englobe également le regard de coulée.

Dans certains modes de réalisation, l'enceinte d'isolation est une enveloppe isolante.

Dans certains modes de réalisation, l'enceinte d'isolation est munie d'un dispositif de chauffage, par exemple un cordon chauffant. Ceci permet de réguler la température au sein de l'enceinte d'isolation et donc de placer l'élément de mesure dans des conditions de températures favorables.

Dans certains modes de réalisation, l'enceinte d'isolation est munie d'une sonde de température. Ceci rend possible une régulation thermique active au sein de l'enceinte d'isolation, le dispositif de chauffage pouvant en particulier être commandé en fonction de la température mesurée par la sonde de température.

Dans certains modes de réalisation, la barrière technique comprend un dispositif de dégazage prévue en amont de l'élément de mesure. Il peut notamment s'agir d'une vanne de purge positionnée en point haut de la circulation. Un tel dispositif de dégazage permet d'évacuer d'éventuelles bulles ou poches de gaz présentes dans la matière à dépoter qui pourraient altérer le fonctionnement de l'élément de mesure.

Dans certains modes de réalisation, le module de validation comprend un module d'identification possédant une base de données de paramètres théoriques pour un ensemble de différentes matières et le module d'identification est configuré pour identifier la matière à dépoter en comparant au moins un paramètre mesuré par l'élément de mesure avec les paramètres théoriques de la base de données. Ainsi, outre la simple comparaison avec la matière attendue pour le dépotage, le module de validation permet, grâce à ce module d'identification, d'identifier la matière à dépoter si cette dernière est incluse dans la base de données.

Dans certains modes de réalisation, le module de validation comprend un module de comparaison configuré pour comparer une signature d'identification de la matière à dépoter fournie par le module d'identification avec une signature préenregistrée de la matière attendue pour le dépotage. Ceci est notamment utile lorsque le module d'identification et le module de comparaison ne sont pas implémentés dans la même unité de calcul, par exemple lorsque le module d'identification est inclus dans l'élément de mesure et que le module de comparaison est inclus dans un automate. Dans un tel cas, l'automate peut se contenter d'effectuer la comparaison des signatures, ces dernières pouvant notamment prendre la forme d'une valeur décimale codée binaire (DCB), sur 4 bits par exemple.

Dans certains modes de réalisation, le module de validation est configuré pour déterminer si la matière à dépoter correspond à la matière attendue pour le dépotage au moins avant le début du dépotage. On interdit ainsi tout début de dépotage et donc tout début de mélange si la matière à dépoter n'est pas celle attendue.

Dans certains modes de réalisation, le module de validation est configuré pour déterminer si la matière à dépoter correspond à la matière attendue pour le dépotage en cours de dépotage. On vérifie ainsi que la composition de la matière à dépoter n'évolue pas au cours du dépotage, par exemple en cas de présence de plusieurs phases dans le camion de livraison. Cette vérification a lieu de préférence à intervalle régulier,

Dans certains modes de réalisation, le module de validation est configuré en outre pour vérifier si la communication entre l'élément de mesure, le module de validation, le module de commande et/ou l'élément de barrage est bien établie. De cette manière, en cas de défaut de communication, on interdit en tout état de cause le dépotage : on évite ainsi une situation selon laquelle une matière incompatible serait dépotée par erreur en raison d'un signal d'identification corrompu par une mauvaise transmission ou d'une rémanence d'un signal d'identification obsolète.

Dans certains modes de réalisation, l'élément de barrage est une pompe. Ainsi, lorsque la pompe est à l'arrêt, elle empêche l'écoulement de la matière à dépoter ; à l'inverse, lorsqu'elle est en fonctionnement, elle entraîne la matière à dépoter et réalise ainsi le dépotage.

Dans certains modes de réalisation, l'élément de barrage est une vanne, par exemple du type électromécanique. Ainsi, lorsqu'elle est fermée, elle empêche l'écoulement de la matière à dépoter ; à l'inverse, lorsqu'elle est ouverte, elle permet l'écoulement de la matière à dépoter à travers la pompe de dépotage.

Dans certains modes de réalisation, la barrière technique comprend un module de communication configuré pour transmettre à un poste de supervision des données relatives au fonctionnement de la barrière technique et/ou au déroulement du dépotage.

Dans certains modes de réalisation, la barrière technique comprend un deuxième élément de mesure configuré pour mesurer au moins un paramètre de la matière à dépoter, un deuxième module de validation configuré pour comparer au moins un paramètre mesuré par ledit deuxième élément de mesure avec un paramètre théorique préenregistré pour la matière attendue pour le dépotage, et déterminer, sur la base de cette comparaison, si la matière à dépoter correspond à la matière attendue pour le dépotage, et le module de commande est configuré pour commander l'élément de barrage en fonction des résultats des déterminations menées par chacun des modules de validation. Une telle redondance permet de renforcer la fiabilité de la barrière technique et donc la sécurité du site et de ses personnels. Le deuxième élément de mesure peut être identique au premier élément de mesure ; à l'inverse, il peut également mesurer un paramètre différent de celui mesuré par le premier élément de mesure.

Le présent exposé concerne également un procédé de sécurisation de dépotage, comprenant les étapes suivantes : mettre en place un élément de barrage entre une cuve amont contenant la matière à dépoter et une cuve aval destinée à recevoir la matière à dépoter, l'élément de barrage étant configuré pour bloquer ou laisser passer la matière à dépoter en fonction de la commande qu'on lui adresse ; mesurer un paramètre de la matière à dépoter en amont de l'élément de barrage ; comparer ce paramètre mesuré avec un paramètre théorique de la matière attendue pour le dépotage et en déduire si la matière à dépoter correspond à la matière attendue pour le dépotage ; dans le cas où il est déterminé que la matière à dépoter correspond à la matière attendue pour le dépotage, commander l'élément de barrage pour laisser passer la matière à dépoter ; dans le cas où il est déterminé que la matière à dépoter ne correspond pas à la matière attendue pour le dépotage, commander l'élément de barrage pour bloquer la matière à dépoter.

De manière analogue à ce qui a été présenté ci-dessus, un tel procédé permet de sécuriser un dépotage en vérifiant si la matière à dépoter correspond bien à la matière attendue et en interdisant le dépotage dans le cas contraire.

Naturellement, ce procédé peut comporter d'autres étapes ou spécificités analogues à ce qui a été présenté ci-dessus au sujet de la barrière technique.

Les caractéristiques et avantages précités, ainsi que d'autres, apparaîtront à la lecture de la description détaillée qui suit, d'exemples de réalisation de la barrière technique proposée. Cette description détaillée fait référence aux dessins annexés.

### BREVE DESCRIPTION DES DESSINS

Les dessins annexés sont schématiques et visent avant tout à illustrer les principes de l'invention.

Sur ces dessins, d'une figure (FIG) à l'autre, des éléments (ou parties d'élément) identiques sont repérés par les mêmes signes de référence. En outre, des éléments (ou parties d'élément) appartenant à des exemples de réalisation différents mais ayant une fonction analogue sont repérés sur les figures par des références numériques incrémentées de 100, 200, etc.
La FIG 1 est un schéma de principe d'une barrière technique selon un premier exemple de réalisation.
La FIG 2 est une vue en perspective d'un regard de coulée.
La FIG 3 est un exemple de spectre Raman.
La FIG 4 est un schéma de principe d'une barrière technique selon un deuxième exemple de réalisation.
La FIG 5 est un schéma de principe d'une barrière technique selon un troisième exemple de réalisation.
La FIG 6 est une vue en perspective d'un regard de coulée selon une variante.

### DESCRIPTION DETAILLEE D'EXEMPLE(S) DE REALISATION

Afin de rendre plus concrète l'invention, des exemples de barrière techniques sont décrits en détail ci-après, en référence aux dessins annexés. Il est rappelé que l'invention ne se limite pas à ces exemples.

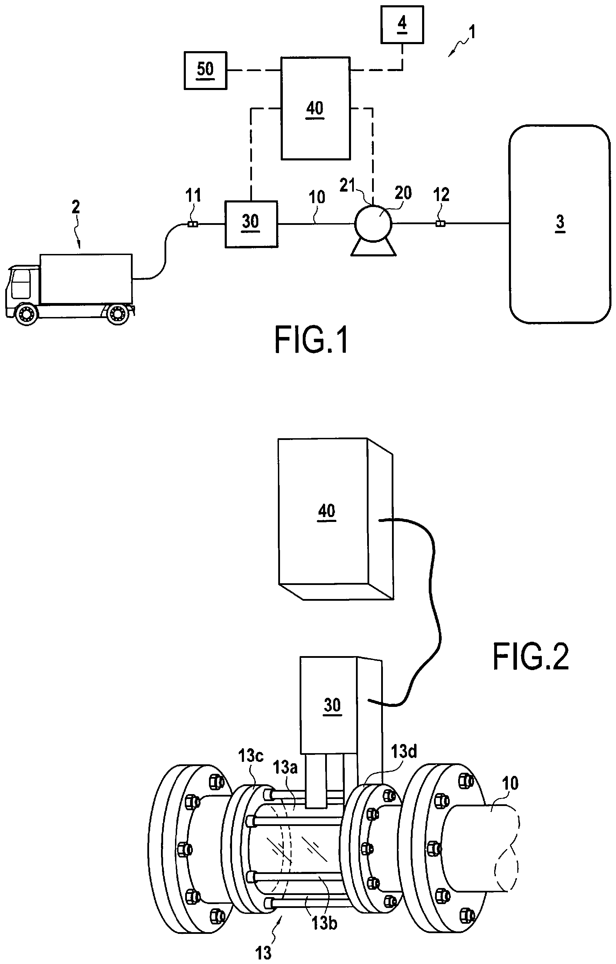

La FIG 1 représente un schéma de principe d'un premier exemple de réalisation d'une barrière technique 1 prévue pour sécuriser le dépotage d'une matière à dépoter depuis une cuve amont, ici un camion 2, vers une cuve aval 3.

La barrière technique 1 comprend un conduit 10, muni d'un raccord amont 11 et d'un raccord aval 12, sur lequel sont montés une pompe 20 et un analyseur en ligne 30 prévu en amont de la pompe 20.

Cette pompe 20 possède une entrée de commande 21 et est configurée pour fonctionner, et donc pour entraîner la matière à dépoter d'amont en aval dans la barrière technique 1, lorsqu'un signal de commande est envoyé à son entrée de commande 21 et pour s'arrêter et rester immobile, et donc pour bloquer l'écoulement de la matière à dépoter dans la barrière technique 1, lorsqu'aucun signal de commande ne parvient à son entrée de commande 21.

L'analyseur en ligne 30 est monté pour sa part sur un regard de coulée 13 du conduit 10. Ce regard de coulée 13, visible sur la FIG 2, comprend un tronçon de conduit 13a totalement transparent, réalisé ici en verre. Il comprend également une pluralité de barres 13b s'étendant entre les brides amont 13c et aval 13d du tronçon de conduit transparent 13a, coaxialement et autour de ce dernier, dans le but d'assurer la tenue mécanique du regard de coulée 13.

L'analyseur en ligne 30 est un spectromètre Raman dont la lentille est plaquée contre la paroi transparente 13a du regard de coulée 13. Ce spectromètre Raman 30 est ainsi capable de mesurer le spectre Raman de la matière s'écoulant dans le regard de coulée 13.

La spectroscopie Raman consiste à envoyer une lumière monochromatique sur l'échantillon et à analyser la lumière diffusée en mesurant le décalage en fréquence causé par l'échange d'énergie entre le rayon lumineux et la matière analysée (effet Raman).

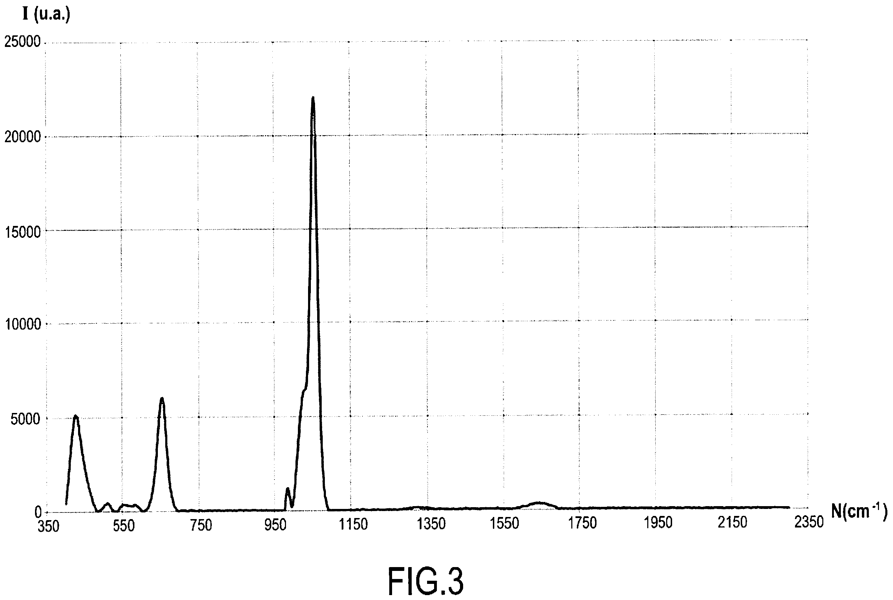

Le spectromètre Raman 30 permet ainsi d'obtenir des spectres tel celui de la FIG 3, correspondant au spectre du bisulfite de sodium, dans lequel l'intensité lumineuse I de la lumière diffusée, exprimée en unités arbitraires (u.a.), est donnée pour chaque nombre d'onde N, exprimés en cm⁻¹, c'est-à-dire pour chaque décalage Raman. Or, chaque substance chimique active en Raman possède un spectre Raman qui lui est propre et qui permet de l'identifier.

A cette fin, le spectromètre Raman 30 est muni en outre d'une base de données répertoriant les spectres Raman de nombreuses substances chimiques et incluant au moins le spectre de la matière attendue pour le dépotage, c'est-à-dire généralement la matière de la cuve aval 3. Le spectromètre Raman 30 comprend de plus un module d'identification capable de comparer le spectre mesuré avec les spectres de sa base de donnée afin d'identifier la matière présente dans le regard de coulée 13.

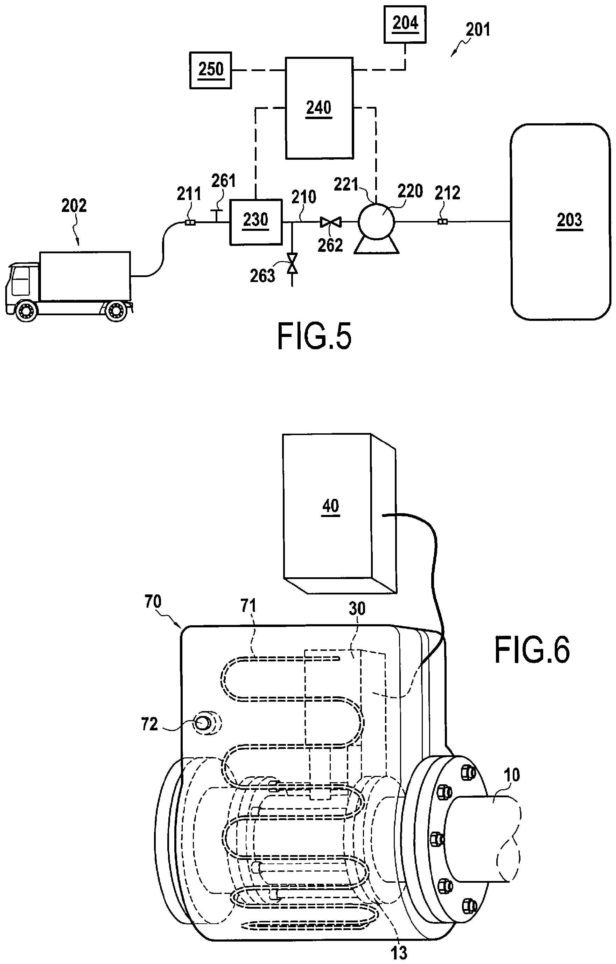

Dans une variante de réalisation représentée en FIG 6, le regard de coulée 13 et l'analyseur en ligne 30 sont enveloppés dans une enveloppe isolante 70, isolant thermiquement le regard de coulée 13 et l'analyseur en ligne 30 par rapport au milieu extérieur.

Cette enveloppe isolante 70 est munie d'un cordon chauffant 71 et d'une sonde de température 72 permettant de mesurer la température au sein de l'enveloppe isolante 70. Une deuxième sonde de température peut également être prévue à proximité de la conduite 10, à l'extérieur de l'enveloppe isolante 70, afin de mesurer la température du milieu extérieur.

L'automate 40 est configuré pour commander le cordon chauffant 71 en fonction des mesures de température réalisées par les sondes de températures 72 de manière à réguler la température au sein de l'enveloppe isolante 70. Ainsi, dans cet exemple, l'automate commande le démarrage du chauffage lorsque la température intérieure descend en dessous d'une température seuil, par exemple 10°C, et commande l'arrêt du chauffage lorsque la température intérieure dépasse une température plafond, par exemple 14°C. Une condition d'arrêt temporisée peut également être prévue en tout état de cause, par exemple un arrêt automatique du chauffage à l'issue de 30 minutes de chauffage ininterrompu.

La barrière technique 1 comprend en outre un automate industriel 40 connecté d'une part à l'analyseur en ligne 30 (éventuellement à travers une interface électronique), d'autre part à l'entrée de commande 21 de la pompe 20 et comprenant des moyens de calcul logique. Il est également connecté à un pupitre de contrôle 50 muni de boutons et de voyants de contrôle ainsi qu'à un poste de supervision distant 4.

Le fonctionnement de la barrière technique va maintenant être décrit en détail.

Lors de la préparation d'un dépotage, la barrière technique 1 est connectée d'une part à la cuve aval 3 grâce à son raccord aval 12 et d'autre part à la cuve amont 2 grâce à son raccord amont 11. Toutefois, dans certains cas, sur un site industriel par exemple, la barrière technique 1 pourrait être prévue de manière permanente sur une cuve 3 donnée, notamment lorsque celui-ci a vocation à toujours contenir la même matière : dans un tel cas, chaque cuve 3 du site pourrait être équipée d'une barrière technique 1 dédiée.

Une fois la barrière technique 1 ainsi connectée, la matière à dépoter prévue dans la cuve amont 2 commence à s'écouler de manière gravitaire dans la barrière 1, remplissant notamment le regard de coulée 13, jusqu'à être arrêtée par la pompe 20 initialement à l'arrêt. Une vanne manuelle intermédiaire peut également être prévue entre le regard de coulée 13 et la pompe 20.

Un opérateur peut alors lancer l'opération de dépotage en appuyant sur un bouton de lancement du pupitre de contrôle 50. Sur la base de ce signal, l'automate 40 lance tout d'abord un auto-test de communication avec l'analyseur 30 (ou son interface lorsqu'une telle interface existe). Si ce test de communication est positif, l'automate 40 envoie alors un signal de commande à l'analyseur 30 lui demandant de lancer l'analyse de la matière à dépoter ; dans le cas contraire, l'automate 40 envoie un signal au pupitre de contrôle 50 pour allumer un voyant de défaut. En cas de défaut, l'opérateur doit vérifier la connexion et le bon fonctionnement de l'analyseur 30 puis appuyer sur un bouton d'effacement de défaut du pupitre de contrôle 50 avant de relancer l'opération de dépotage. Dans un autre exemple, il pourrait être prévu que l'opérateur ne puisse pas acquitter le défaut, seule une intervention dans l'automate par un automaticien pouvant alors l'acquitter après vérification en particulier du câblage et des entrées-sorties de l'analyseur et de l'automate.

Lorsque l'analyseur 30 reçoit le signal de lancement de l'analyse de la part de l'automate 40, il mesure le spectre Raman de la matière à dépoter présente dans le regard de coulée 13 puis tente de l'identifier en le comparant avec les spectres de sa base de données. En cas d'identification, l'analyseur 30, éventuellement via son interface, envoie un signal à l'automate formant signature de la matière identifiée. Dans le présent exemple, cette signature est codée en binaire sur quatre bits à l'aide de quatre fils connectées à des entrées tout-ou-rien de l'automate 40. En outre, une signature spécifique peut être prévue lorsque l'analyseur 30 n'est pas parvenu à identifier la matière ou lorsqu'il a rencontré un défaut.

L'automate 40 compare alors la signature ainsi transmise par l'analyseur 30 avec la signature de la matière attendue pour le dépotage préenregistrée dans l'automate 40. Lorsque l'automate 40 détermine qu'il y a correspondance, il envoie un signal de commande à l'entrée de commande 21 de la pompe 20. Dans le cas contraire, il envoie un signal au pupitre de contrôle 50 pour allumer un voyant de défaut. En cas de défaut, l'opérateur doit vérifier que la bonne cuve amont 2 a bien été connectée à la barrière 1 ou que la matière contenue dans la cuve amont 2 est bien celle attendue puis appuyer sur un bouton d'effacement de défaut du pupitre de contrôle 50 avant de relancer l'opération de dépotage. Dans un autre exemple, il pourrait être prévu que l'opérateur ne puisse pas acquitter le défaut, seule une intervention dans l'automate par un automaticien pouvant alors l'acquitter. En cas de défaut produit non-conforme, le camion est renvoyé. En cas de défaut absence d'analyse, un diagnostic est établi et chaque élément constitutif est vérifié et des pièces de rechange (câble, analyseur, interface...) sont mises en oeuvre en fonction du diagnostic.

Lorsque la pompe 20 reçoit le signal de commande de l'automate 40, elle démarre et entraîne ainsi le transfert de la matière à dépoter de la cuve amont 2 vers la cuve aval 3.

Toutefois, tant que le dépotage n'est pas terminé ou qu'il n'a pas été interrompu par l'opérateur à l'aide du bouton d'arrêt du pupitre de contrôle 50, l'automate relance régulièrement l'auto-test de communication et l'analyse de la matière à dépoter. En cas de défaut de communication ou de défaut de conformité de la matière à dépoter, l'automate cesse immédiatement d'envoyer un signal de commande à l'entrée de commande 21 de la pompe 20 et déclenche l'allumage d'un voyant de défaut sur le pupitre de contrôle 50 : la pompe 20 cesse dès lors de fonctionner et bloquer à nouveau l'écoulement de la matière à dépoter.

En outre, durant toute sa période de fonctionnement, l'automate transmet au poste de supervision 4 des informations sur le déroulement du dépotage, notamment l'état des différents signaux de commande et de défaut et la signature d'identification de la matière circulant dans la barrière technique 1.

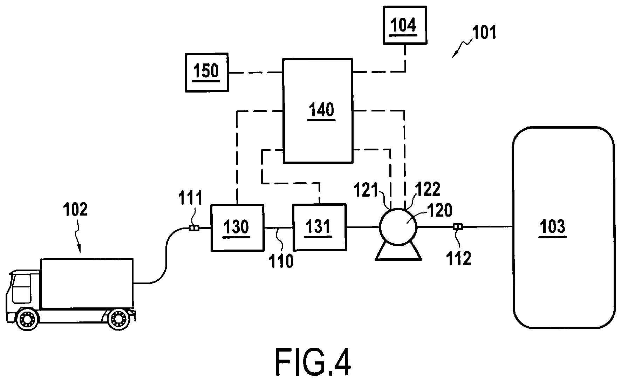

La FIG 4 représente un schéma de principe d'un deuxième exemple de réalisation d'une barrière technique 101 prévue pour sécuriser le dépotage d'une matière à dépoter depuis une cuve amont, ici un camion 102, vers une cuve aval 103. La structure et le fonctionnement de cette barrière technique 101 sont tout à fait analogues à ceux de la barrière technique 1 du premier exemple de telle sorte que seules les différences avec ce premier exemple seront décrites.

Dans ce deuxième exemple, la pompe 120 comprend une première entrée de commande 121 et une deuxième entrée de commande 122. Elle est ainsi configurée pour ne fonctionner que lorsque des signaux de commande parviennent à la fois à la première entrée de commande 121 et à la deuxième entrée de commande 122.

De plus, la barrière technique 101 comporte désormais un premier analyseur en ligne 130 et un deuxième analyseur en ligne 131, tous deux analogues à l'analyseur en ligne 30 du premier exemple, montés l'un après l'autre sur des regards de coulées, ou un regard de coulée unique, du conduit 110 de la barrière technique 101.

Dès lors, dans ce deuxième exemple, une fois l'opération de dépotage lancée, l'automate 140 va vérifier la communication avec chacun des analyseurs 130, 131 puis demander à chacun d'entre eux de lancer une analyse de la matière à dépoter : le résultat issu du premier analyseur 130 détermine si l'automate 140 envoie un premier signal de commande à la première entrée 121 de la pompe 120 tandis que le résultat du deuxième analyseur détermine, de manière complètement indépendante, si l'automate 140 envoie un deuxième signal de commande à la deuxième entrée de commande 122 de la pompe 120. Dès lors, la pompe 120 ne démarre, et donc le dépotage n'a lieu, que si les deux analyseurs 130 et 131 déterminent de manière indépendante que la matière à dépoter est bien la matière attendue pour le dépotage.

Dans une variante de ce deuxième exemple de réalisation, la barrière technique pourrait comprendre deux automates indépendants, le premier connecté au premier analyseur et à la première entrée de commande de la pompe, et le deuxième connecté au deuxième analyseur et à la deuxième entrée de commande de la pompe.

La FIG 5 représente un schéma de principe d'un troisième exemple de réalisation d'une barrière technique 201 prévue pour sécuriser le dépotage d'une matière à dépoter depuis une cuve amont, ici un camion 202, vers une cuve aval 203. La structure et le fonctionnement de cette barrière technique 201 sont tout à fait analogues à ceux de la barrière technique 1 du premier exemple de telle sorte que seules les différences avec ce premier exemple seront décrites. Il va toutefois de soi que les enseignements de ce troisième exemple de réalisation sont compatibles avec le deuxième exemple de réalisation.

Dans ce troisième exemple, une vanne de dégazage 261 est prévue entre le raccord amont 211 et le regard de coulée portant l'analyseur en ligne 230. Cette vanne de dégazage est prévue plus précisément au point haut de la conduite 210 de manière à évacuer le gaz éventuellement présent dans la matière à dépoter avant son entrée dans le regard de coulée.

De plus, une vanne d'isolement 262 est prévue entre le regard de coulée portant l'analyseur en ligne 230 et la pompe 220. Une vanne de vidange 263 est par ailleurs prévue en aval du regard de coulée portant l'analyseur en ligne 230 et en amont de la vanne d'isolement 262. De cette manière, il est possible de vidanger le regard de coulée en fermant la vanne d'isolement 262 et en ouvrant la vanne de vidange 263, la vidange s'effectuant alors de manière gravitaire.

La vanne d'isolement 262 est équipée d'un détecteur de fin de course permettant de renvoyer à l'automate 240 l'information sur la position ouverte ou fermée de la vanne d'isolement 262.

Dans cet exemple, une vidange systématique du regard de coulée et un test à vide sont réalisés après chaque opération de dépotage. Ainsi, une fois la fin de l'opération de dépotage signifiée à l'automate 240, l'automate commande l'arrêt de la pompe 220 et la fermeture de la vanne d'isolement 262 puis commande l'ouverture de la vanne de vidange 263 de manière à entraîner la vidange gravitaire du regard de coulée.

Dans cet exemple, les vannes d'isolement 262 et de vidange 263 sont commandées par l'automate 240 ; toutefois, dans d'autres exemples, elles pourraient être manuelles.

A l'issue d'une durée prédéterminée suffisante pour assurer la vidange complète du regard de coulée, par exemple 30 minutes, l'analyseur en ligne 230 réalise un test à vide dans le regard de coulée ainsi vidangé.

Si le regard est effectivement vide, l'analyseur 230 détectera une absence de matière dans le regard et transmettra à l'automate 240 une signature correspondant à ce cas de figure. L'automate 240 confirmera ainsi le succès de la vidange, ce qui est une condition préalable au dépotage suivant.

A l'inverse, si un résidu de matière à dépoter reste présent dans le regard, l'analyseur 230 transmettra à l'automate 240 la signature de la matière identifiée. L'automate 240 allumera alors un voyant de défaut et interdira tout nouveau dépotage à moins qu'une nouvelle opération de vidange soit réalisée avec succès.

Bien que la présente invention ait été décrite en se référant à des exemples de réalisation spécifiques, il est évident que des modifications et des changements peuvent être effectués sur ces exemples sans sortir de la portée générale de l'invention telle que définie par les revendications. En particulier, des caractéristiques individuelles des différents modes de réalisation illustrés/mentionnés peuvent être combinées dans des modes de réalisation additionnels. Par conséquent, la description et les dessins doivent être considérés dans un sens illustratif plutôt que restrictif.

Il est également évident que toutes les caractéristiques décrites en référence à un procédé sont transposables, seules ou en combinaison, à un dispositif, et inversement, toutes les caractéristiques décrites en référence à un dispositif sont transposables, seules ou en combinaison, à un procédé.

## Revendications

1. Barrière technique (1) pour la sécurisation de dépotages, comprenant
un élément de barrage (20) configuré pour bloquer ou laisser passer une matière à dépoter en fonction d'une commande qui lui est adressée,
un élément de mesure (30) prévu en amont de l'élément de barrage (20) et configuré pour mesurer au moins un paramètre de la matière à dépoter,
un module de validation (30-40) configuré pour comparer au moins un paramètre mesuré par ledit élément de mesure (30) avec un paramètre théorique préenregistré pour la matière attendue pour le dépotage, et déterminer, sur la base de cette comparaison, si la matière à dépoter correspond à la matière attendue pour le dépotage, et
un module de commande (40) configuré pour commander l'élément de barrage (20) en fonction des résultats de cette détermination,
**caractérisé en ce que** l'élément de mesure est un analyseur en ligne (30) configuré pour mesurer au moins un paramètre de la matière à dépoter sans prélèvement de matière.

2. Barrière technique (1) selon la revendication 1, dans laquelle ledit analyseur en ligne (30) est configuré pour mesurer au moins un paramètre de la matière à dépoter sans contact avec ladite matière à dépoter.

3. Barrière technique (1) selon la revendication 2, dans laquelle l'élément de mesure est un spectromètre (30), de préférence de type Raman.

4. Barrière technique (1) selon l'une quelconque des revendications 1 à 3, comprenant un regard de coulée (13) configuré pour recevoir la matière à dépoter en amont de l'élément de barrage (20), le regard de coulée (13) possédant au moins une fenêtre transparente (13a) au niveau de laquelle est prévu l'élément de mesure (30).

5. Barrière technique (1) selon la revendication 4, comprenant une vanne de vidange (263) prévue en aval du regard de coulée et en amont de l'élément de barrage (220).

6. Barrière technique (1) selon l'une quelconque des revendications 1 à 5, comprenant une enceinte d'isolation thermique (70) englobant au moins l'élément de mesure (30) afin de l'isoler du milieu environnant.

7. Barrière technique (1) selon la revendication 6, dans laquelle l'enceinte d'isolation thermique (70) est munie d'un dispositif de chauffage (71), par exemple un cordon chauffant.

8. Barrière technique (1) selon l'une quelconque des revendications 1 à 7, comprenant un dispositif de dégazage (261) prévu en amont de l'élément de mesure (230).

9. Barrière technique (1) selon l'une quelconque des revendications 1 à 8, dans laquelle le module de validation comprend un module d'identification possédant une base de données de paramètres théoriques pour un ensemble de différentes matières, et
dans laquelle le module d'identification est configuré pour identifier la matière à dépoter en comparant au moins un paramètre mesuré par l'élément de mesure (30) avec les paramètres théoriques de la base de données.

10. Barrière technique (1) selon la revendication 9, dans laquelle le module de validation comprend un module de comparaison (40) configuré pour comparer une signature d'identification de la matière à dépoter fournie par le module d'identification avec une signature préenregistrée de la matière attendue pour le dépotage.

11. Barrière technique (1) selon l'une quelconque des revendications 1 à 10, dans laquelle le module de validation (30-40) est configuré pour déterminer si la matière à dépoter correspond à la matière attendue pour le dépotage au moins avant le début du dépotage et éventuellement en cours de dépotage.

12. Barrière technique (1) selon l'une quelconque des revendications 1 à 11, dans laquelle l'élément de barrage est une pompe (20).

13. Barrière technique (1) selon l'une quelconque des revendications 1 à 12, dans laquelle l'élément de barrage est une vanne (20).

14. Barrière technique (1) selon l'une quelconque des revendications 1 à 13, comprenant un module de communication (40) configuré pour transmettre à un poste de supervision (4) des données relatives au fonctionnement de la barrière technique (1) et/ou au déroulement du dépotage.

15. Procédé de sécurisation de dépotage, comprenant les étapes suivantes :
mettre en place un élément de barrage (20) entre une cuve amont (2) contenant la matière à dépoter et une cuve aval (3) destinée à recevoir la matière à dépoter, l'élément de barrage (20) étant configuré pour bloquer ou laisser passer la matière à dépoter en fonction de la commande qu'on lui adresse,
mesurer un paramètre de la matière à dépoter en amont de l'élément de barrage (20),
comparer ce paramètre mesuré avec un paramètre théorique de la matière attendue pour le dépotage et en déduire si la matière à dépoter correspond à la matière attendue pour le dépotage,
dans le cas où il est déterminé que la matière à dépoter correspond à la matière attendue pour le dépotage, commander l'élément de barrage (20) pour laisser passer la matière à dépoter,
dans le cas où il est déterminé que la matière à dépoter ne correspond pas à la matière attendue pour le dépotage, commander l'élément de barrage (20) pour bloquer la matière à dépoter,
**caractérisé en ce que** lequel ledit paramètre de la matière à dépoter est mesuré à l'aide d'un analyseur en ligne (30) configuré pour mesurer au moins un paramètre de la matière à dépoter sans prélèvement de matière.

## Patentansprüche

1. Technische Barriere (1) zum Sichern von Entladungen, die umfasst
ein Sperrelement (20), das ausgelegt ist, um ein zu entladendes Material in Abhängigkeit von einem Befehl, der ihm geschickt wurde, zu blockieren oder passieren zu lassen,
ein Messelement (30), das dem Sperrelement (20) vorgelagert vorgesehen und ausgelegt ist, um mindestens einen Parameter des zu entladenden Materials zu messen,
ein Validierungsmodul (30-40), das ausgelegt ist, um mindestens einen von dem Messelement (30) gemessenen Parameter mit einem für das für das Entladen erwartete Material vorgespeicherten theoretischen Parameter zu vergleichen und auf der Basis dieses Vergleichs zu bestimmen, ob das zu entladende Material dem für das Entladen erwarteten Material entspricht, und
ein Steuermodul (40), das ausgelegt ist, um das Sperrelement (20) in Abhängigkeit von den Ergebnissen dieser Bestimmung zu steuern,
**dadurch gekennzeichnet, dass** das Messelement ein Inline-Analysator (30) ist, der ausgelegt ist, um mindestens einen Parameter des zu entladenden Materials ohne Materialentnahme zu messen.

2. Technische Barriere (1) nach Anspruch 1, wobei der Inline-Analysator (30) ausgelegt ist, um mindestens einen Parameter des zu entladenden Materials ohne Kontakt mit dem zu entladenden Material zu messen.

3. Technische Barriere (1) nach Anspruch 2, wobei das Messelement ein Spektrometer (30), vorzugsweise vom Typ Raman, ist.

4. Technische Barriere (1) nach einem der Ansprüche 1 bis 3, die ein Durchfluss-Schauglas (13) umfasst, das ausgelegt ist, um das zu entladende Material dem Sperrelement (20) vorgelagert aufzunehmen, wobei das Durchfluss-Schauglas (13) mindestens ein transparentes Fenster (13a) besitzt, in dessen Bereich das Messelement (30) vorgesehen ist.

5. Technische Barriere (1) nach Anspruch 4, die ein Ablassventil (263) umfasst, das dem Durchfluss-Schauglas nachgelagert und dem Sperrelement (220) vorgelagert vorgesehen ist.

6. Technische Barriere (1) nach einem der Ansprüche 1 bis 5, die einen thermisch isolierenden Behälter (70) umfasst, der mindestens das Messelement (30) umschließt, um es von der Umgebung zu isolieren.

7. Technische Barriere (1) nach Anspruch 6, wobei der thermische isolierende Behälter (70) mit einer Heizvorrichtung (71), beispielsweise einem Heizkabel, versehen ist.

8. Technische Barriere (1) nach einem der Ansprüche 1 bis 7, die eine Entgasungsvorrichtung (261) umfasst, die dem Messelement (230) vorgelagert vorgesehen ist.

9. Technische Barriere (1) nach einem der Ansprüche 1 bis 8, wobei das Validierungsmodul ein Identifikationsmodul umfasst, das eine Datenbank mit theoretischen Parametern für eine Gesamtheit unterschiedlicher Materialien besitzt, und
wobei das Identifikationsmodul ausgelegt ist, um das zu entladende Material durch Vergleichen mindestens eines vom Messelement (30) gemessenen Parameters mit den theoretischen Parametern der Datenbank zu identifizieren.

10. Technische Barriere (1) nach Anspruch 9, wobei das Validierungsmodul ein Vergleichsmodul (40) umfasst, das ausgelegt ist, um eine Identifikationssignatur des zu entladenden Materials, die von dem Identifikationsmodul bereitgestellt wird, mit einer vorgespeicherten Signatur des für das Entladen erwarteten Materials zu vergleichen.

11. Technische Barriere (1) nach einem der Ansprüche 1 bis 10, wobei das Validierungsmodul (30-40) ausgelegt ist, um zu bestimmen, ob das zu entladende Material dem für das Entladen erwarteten Material mindestens vor dem Beginn des Entladens und gegebenenfalls während des Entladens entspricht.

12. Technische Barriere (1) nach einem der Ansprüche 1 bis 11, wobei das Sperrelement eine Pumpe (20) ist.

13. Technische Barriere (1) nach einem der Ansprüche 1 bis 12, wobei das Sperrelement eine Pumpe (20) ist.

14. Technische Barriere nach einem der Ansprüche 1 bis 13, die ein Kommunikationsmodul (40) umfasst, das ausgelegt ist, um an einen Kontrollposten (4) Daten zu übertragen, die sich auf den Betrieb der technischen Barriere (1) und/oder den Ablauf des Entladens beziehen.

15. Verfahren zur Sicherung des Entladens, das die folgenden Schritte umfasst:
Platzieren eines Sperrelements (20) zwischen einem vorgelagerten Tank (2), der das zu entladende Material enthält, und einem nachgelagerten Tank (3) zur Aufnahme des zu entladenden Materials, wobei das Sperrelement (20) ausgelegt ist, um das zu entladende Material in Abhängigkeit von einem ihm übermittelten Befehl zu blockieren oder passieren zu lassen,
Messen eines Parameters des dem Sperrelement (20) vorgelagerten zu entladenden Materials,
Vergleichen dieses gemessenen Parameters mit einem theoretischen Parameter des für das Entladen erwarteten Materials und Ableiten daraus, ob das zu entladende Material dem für das Entladen erwarteten Material entspricht,
wenn festgestellt wird, dass das zu entladende Material dem für das Entladen erwarteten Material entspricht, Steuern an das Sperrelement (20), das zu entladende Material passieren zu lassen,
wenn festgestellt wird, dass das zu entladende Material nicht dem für das Entladen erwarteten Material entspricht, Steuern an das Sperrelement (20), das zu entladende Material zu blockieren,
**dadurch gekennzeichnet, dass** der Parameter des zu entladenden Materials mit Hilfe eines Inline-Analysators (30) gemessen wird, der ausgelegt ist, um mindestens einen Parameter des zu entladenden Materials ohne Materialentnahme zu messen.

## Claims

1. A technical barrier (1) for ensuring the security of unloading operations, comprising:
a barrier element (20) configured to block or let pass a material to be unloaded according to a command sent thereto,
a measuring element (30) provided upstream of the barrier element (20) and configured to measure at least one parameter of the material to be unloaded,
a validation module (30-40) configured to compare at least one parameter measured by said measuring element (30) with a prerecorded theoretical parameter for the material expected for the unloading, and to determine, based on this comparison, whether the material to be unloaded corresponds to the material expected for the unloading, and
a control module (40) configured to control the barrier element (20) according to the results of this determination,
**characterized in that** the measuring element is an in-line analyzer (30) configured to measure at least one parameter of the material to be unloaded without sampling of material.

2. The technical barrier (1) according to claim 1, wherein said in-line analyzer (30) is configured to measure at least one parameter of the material to be unloaded without contact with said material to be unloaded.

3. The technical barrier (1) according to claim 2, wherein the measuring element is a spectrometer (30), preferably of the Raman type.

4. The technical barrier (1) according to any one of claims 1 to 3, comprising a sight glass (13) configured to receive the material to be unloaded upstream of the barrier element (20), the sight glass (13) having at least one transparent window (13a) at which the measuring element (30) is provided.

5. The technical barrier (1) according to claim 4, comprising a drain valve (263) provided downstream of the sight glass and upstream of the barrier element (220).

6. The technical barrier (1) according to any one of claims 1 to 5, comprising a thermal insulation enclosure (70) encompassing at least the measuring element (30) in order to insulate it from the surrounding environment.

7. The technical barrier (1) according to claim 6, wherein the thermal insulation enclosure (70) is provided with a heating device (71), for example a heating cord.

8. The technical barrier (1) according to any one of claims 1 to 7, comprising a degassing device (261) provided upstream of the measuring element (230).

9. The technical barrier (1) according to any one of claims 1 to 8, wherein the validation module comprises an identification module having a database of theoretical parameters for a set of different materials, and
wherein the identification module is configured to identify the material to be unloaded by comparing at least one parameter measured by the measuring element (30) with the theoretical parameters of the database.

10. The technical barrier (1) according to claim 9, wherein the validation module comprises a comparison module (40) configured to compare a signature of identification of the material to be unloaded provided by the identification module with a pre-recorded signature of the material expected for the unloading.

11. The technical barrier (1) according to any one of claims 1 to 10, wherein the validation module (30-40) is configured to determine whether the material to be unloaded corresponds to the material expected for the unloading at least before the start of the unloading and possibly during the unloading.

12. The technical barrier (1) according to any one of claims 1 to 11, wherein the barrier element is a pump (20).

13. The technical barrier (1) according to any one of claims 1 to 12, wherein the barrier element is a valve (20).

14. The technical barrier (1) according to any one of claims 1 to 13, comprising a communication module (40) configured to transmit to a station for supervising (4) the data relating to the operation of the technical barrier (1) and /or to the progress of the unloading.

15. A method for ensuring the security of an unloading operation, comprising the following steps:
installing a barrier element (20) between an upstream tank (2) containing the material to be unloaded and a downstream tank (3) intended to receive the material to be unloaded, the barrier element (20) being configured to block or let pass the material to be unloaded according to the command sent thereto,
measuring a parameter of the material to be unloaded upstream of the barrier element (20),
comparing this measured parameter with a theoretical parameter of the material expected for the unloading and deducing therefrom whether the material to be unloaded corresponds to the material expected for the unloading,
in the event that it is determined that the material to be unloaded corresponds to the material expected for the unloading, controlling the barrier element (20) to let pass the material to be unloaded,
in the event that it is determined that the material to be unloaded does not correspond to the material expected for the unloading, controlling the barrier element (20) to block the material to be unloaded,
**characterized in that** said parameter of the material to be unloaded is measured using an in-line analyzer (30) configured to measure at least one parameter of the material to be unloaded without sampling of material.
